(19)

Europäisches
Patentamt

European
Patent Office

Office européen
des brevets

(11) **EP 4 132 343 B1**

(12) **EUROPEAN PATENT SPECIFICATION**

(45) Date of publication and mention
of the grant of the patent:
**13.08.2025 Bulletin 2025/33**

(21) Application number: **20733221.4**

(22) Date of filing: **09.06.2020**

(51) International Patent Classification (IPC):
***A61B 3/10*** *(2006.01)*

(52) Cooperative Patent Classification (CPC):
**A61B 3/10; A61B 3/102**

(86) International application number:
**PCT/EP2020/065940**

(87) International publication number:
**WO 2021/249624 (16.12.2021 Gazette 2021/50)**

(54) **METHOD AND DEVICE FOR FLUORESCENCE LIFETIME MICROSCOPY ON AN EYE**

VERFAHREN UND VORRICHTUNG ZUR FLUORESZENZLEBENSDAUERMIKROSKOPIE AN
EINEM AUGE

PROCÉDÉ ET DISPOSITIF DE MICROSCOPIE DE DURÉE DE VIE DE FLUORESCENCE SUR UN
OEIL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**15.02.2023 Bulletin 2023/07**

(73) Proprietor: **HAAG-STREIT AG
3098 Köniz (CH)**

(72) Inventor: **ROBLEDO, Lucio
3013 Bern (CH)**

(74) Representative: **E. Blum & Co. AG
Franklinturm
Hofwiesenstrasse 349
8050 Zürich (CH)**

(56) References cited:
WO-A1-2008/133693    WO-A1-2015/072964
WO-A1-2018/001838    WO-A1-2019/002256
US-A- 4 569 354      US-A1- 2017 065 179

**Description**

Technical Field

The invention relates to methods and devices for carrying out fluorescence lifetime microscopy on an eye.

Background Art

Numerous techniques have been applied for characterizing the human eye.

One of them is Optical Coherence Tomography (OCT), which allows to locate and characterize structures within the eye, see e.g. EP 3572765.

Fluorescence Lifetime Imaging Microscopy FLIM is one of many other techniques used for characterizing the eye, e.g. the retina, see e.g. WO 2015/072964.

WO 2019/002256 A1 describes a fundus inspection apparatus. It mentions that the fluorescence of the lens interferes with fluorescence measurements of the retina.

Disclosure of the Invention

The problem to be solved by the present invention is to provide higher quality fluorescence lifetime microscopy data for an eye.

This problem is solved by the method and device of the independent claims.

Accordingly, the invention relates to a method for carrying out fluorescence lifetime microscopy on a first structure of an eye, such as the retina. This method comprises at least the following steps:

- Sending, by means of a probe light source, a probe beam into the eye: This is the light beam that will give rise to the fluorescence. It interacts with at least said first structure as well as with a second structure of said eye, such as the lens. The first and said second structure are spaced apart from each other.
- Measuring, by means of a fluorescence detector, time-resolved "raw fluorescence data" returning from the eye: This is the time-resolved fluorescence data including signals from both structures.
- Calculating time-resolved "corrected fluorescence data" for the first structure: This corrected fluorescence data, which more accurately describes the fluorescent response of the first structure, is calculated from the "raw fluorescence data" and from "estimated fluorescence data" originating from the second structure.

This method is based on the understanding that it is possible to account for the influence of the second structure in the overall raw fluorescence data by providing an estimate of its fluorescence and using said estimate to correct the raw fluorescence data.

Advantageously, for good spatial resolution of the first structure, the method comprises the step of performing a first time-resolved fluorescence measurement with the probe beam focused on the first structure. The raw fluorescence data is then derived from this first measurement.

In addition, the estimated fluorescence data may then e.g. be derived from at least one second time-resolved fluorescence measurement with the probe beam focused on the second structure. This is based on the understanding that, by focusing the probe beam on the second structure, the signal will be more sensitive to the second structure than in the first measurement. Hence, the second measurements can be used to derive the estimated fluorescence data for the second structure.

The order of the first and the second time-resolved fluorescence measurement is arbitrary, i.e. the second time-resolved fluorescence measurement may take before or after the first time-resolved fluorescence measurement.

At least one of the two time-resolved fluorescence measurements advantageously includes determining at least one parameter indicative of a decay time of the fluorescence.

The raw fluorescence data can be measured for at least two locations (i.e. for several locations) of the first structure, in particular for different x- and y-locations (with x and y denoting directions perpendicular to the optical axis z of the eye and the microscope device).

In that case, the "estimated fluorescence data" may be one of the following:

a) The estimated fluorescence data may be the same for both locations. In this case, it is assumed that the second structure (such as the lens of the eye) adds substantially the same fluorescence contribution at both measurement locations.

b) The estimated fluorescence data is different between the first and the second location. In this case, it is assumed that the fluorescent properties of the second structure are sufficiently inhomogeneous to warrant a spatially resolved correction.

In case b), spatially resolved estimated fluorescence data may be obtained by performing a plurality of the "second" time-resolved fluorescence measurements with the probe beam focused on different parts of the second structure.

Also in case b), the method may comprise the step of determining the two parts of the second structure that the probe beam interacts with when being focused on the two locations of the first structure. This allows using the estimated fluorescence data attributed to these two (usually different) parts. The determination of the two parts can e.g. be carried out by means of ray tracing calculations.

In an advantageous embodiment, a device equipped for OCT (= Optical Coherence Tomography) and time-resolved fluorescence measurements is used for said fluorescence lifetime microscopy, and the method comprises the step of carrying out OCT measurements on the first and/or second structures with said device. This allows to complement the time-resolved fluorescence measurements with spatially well correlated data on the 3D structure of the eye.

Advantageously, the OCT measurements are carried out by means of a measurement beam collinear to the probe beam used for the fluorescence measurements. This provides an even better spatial correlation between the two types of measurements.

In one embodiment, OCT data obtained from the OCT measurements may be used for calculating the estimated fluorescence data. This allows to take e.g. the geometry of the second structure into account when estimating its fluorescence. For example, at least one of the following parameters of the second structure may be taken into account: its thickness along the axis of the eye, its volume, its extension perpendicular to the axis of the eye, or its position.

In an advantageous embodiment, the first structure is the retina of the eye and/or the second structure is the lens of the eye.

In another embodiment, the second structure may be the retina of the eye and/or the first structure is the lens of the eye.

The invention also relates to a microscope device for carrying out fluorescence lifetime microscopy on a first structure of an eye, comprising at least the following elements:

- A probe light source: The probe light source is adapted and structured to send a probe beam into the eye in order to excite fluorescence therein.
- A fluorescence detector. The fluorescence detector is adapted and structured to measure time-resolved fluorescence data for fluorescent light returning from the eye.
- A control unit adapted to carry out the steps of the method described above.

In a preferred embodiment, the invention also relates to a microscope device for carrying out fluorescence lifetime microscopy of an eye. In this embodiment, the fluorescence detector is adapted and structured to measure time-resolved fluorescence data for fluorescent light returning from the eye and to derive fluorescence lifetime parameters therefrom. The device further comprises:

- An interferometer: The interferometer is adapted and structured to send a measurement beam into the eye and carrying out optical coherence tomography on light of the measurement beam reflected from structures within said eye.

This device allows to measure a combination of time-resolved fluorescence data and OCT data, thereby allowing to accurately relate the two types of data with each other.

Advantageously, the "fluorescence lifetime parameters" include a value indicative of at least one decay time of the fluorescence.

The device may further comprise a beam combiner arranged to collinearly combine the (fluorescence) probe beam and the (interferometric) measurement beam, i.e. to make these two beams spatially concentric and propagating into the same direction. This allows an even better spatial correlation between the two measurement methods.

The OCT measurements allow to measure the distance between the device and at least one part of the eye. In all aspects of the invention, this distance can be used for one or both of the following purposes:

a) To compensate a time offset in the fluorescence data, such as the raw fluorescence data, the estimated fluorescence data, and/or the corrected fluorescence data: This allows to accurately predict the "start time" of the fluorescence data and/or to compensate for changes in the device-eye-distance between consecutive measurements.

b) To enable a fluorescence measurement only if said distance is in a given range. This allows to disable measurements when the distance is inappropriate.

The method may further comprise the steps of measuring said distance for at least two subsequent fluorescence measurements and mutually offsetting, in time, the two fluorescence measurements as a function of the change of said distance in between the two fluorescence measurements.

The part of the eye to be used for the distance measurement is e.g. the first or the second structure of the eye, which allows to obtain the absolute "zero point" of the fluorescence data from the respective structure. If, however, only relative changes of the distance need to be tracked, any part of the eye can be used.

Brief Description of the Drawing

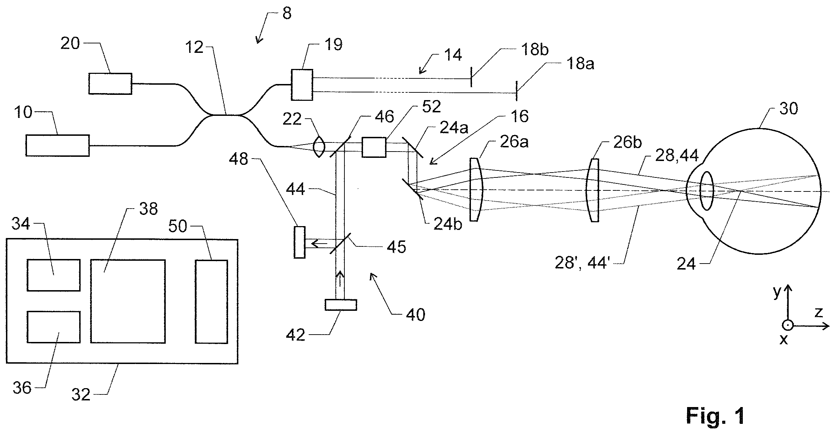

The invention will be better understood and objects other than those set forth above will become apparent when consideration is given to the following detailed description thereof. Such description makes reference to the annexed Fig. 1, which shows the schematic setup of an embodiment of an ophthalmologic microscope device.

Modes for Carrying Out the Invention

*Device Overview*

Fig. 1 shows an example of a device for implementing the present invention. It is an ophthalmologic microscope device equipped to carry out fluorescence lifetime microscopy as well as OCT measurements.

For carrying out OCT measurement, the device comprises an optical coherence tomography interferometer 8.

Interferometer 8 has a light source 10, which, in the present embodiment, is a swept-source light source, i.e. it generates narrowband light that can be adjusted in wavelength.

The light from light source 10 passes a beam splitter 12, in particular a fiber beam splitter, and is sent into two interferometer arms 14, 16.

The first arm is the reference arm 14, which comprises one or more mirrors, in the present case two mirrors 18a, 18b, at one end. Light impinging on the mirrors 18a, 18b is sent back into beam splitter 12 and from there, at least in part, to a light detector 20.

In the shown embodiment, reference arm 14 is provided with an optical switch 19 and, as mentioned, two mirrors 18a, 18b at different positions. Switch 19 can be controlled to send light either to mirror 18a or 18b, with the two settings corresponding to different lengths of reference arm 14. Alternatively, it is e.g. possible to use a single mirror with adjustable position.

By changing the length of reference arm 14, the measurement range of interferometer 8 can be adjusted to measure the anterior or the posterior part of eye 30.

In yet another embodiment, the length of reference arm 14 is not adjustable. In this case, interferometer 8 must have a sufficient range of measurement to scan all parts of the eye that are relevant in the present context, e.g. by using a light source 10 that is sufficiently narrow-width and a detector able to sample the interference signal at sufficiently small wavelength intervals.

The second arm of interferometer 8 is the sample arm 16. It comprises collimation optics 22 for collimating the measurement light coming from beam splitter 12. The light is then fed through two scanner mirrors 24a, 24b and an objective lenses 26a, 26b for generating a measurement beam 28. Depending on the position of the scanner mirrors 24a, 24b, measurement beam 28 can be laterally offset in an x-y-plane perpendicular to the optical axis z of the device.

In the embodiment of Fig. 1, the measurement beam is shown to be focused on the retina of the eye, but it may also be focused on any other part of the eye 30 that is of particular interest, in particular the lens. A focus controller 52 may be provided to change the location of the focus along the optical axis z of the device. Focus controller 52 may e.g. be formed by one or more lenses with adjustable position(s). Alternatively, focus controller 52 may be implemented by an actuator to change the distance between the objective lenses 26a, 26b.

Measurement beam 28 enters eye 30, where it is reflected or scattered by the structures of the eye. Light cast back from such structures is returned to beam splitter 12 and from there, at least in part, to light detector 20, where it can interfere with the light from reference arm 14.

For OCT measurements, the device of Fig. 1 is e.g. operated by recording a plurality of A-scans. For each such A-scan, measurement beam 28 is brought into a desired x- and y-position by means of the scanner mirrors 24a, 24b. Then, the central wavelength of light source 10 is tuned over a given wavelength range, which wavelength range is typically much broader than the spectral width of the light from light source 10. The light at light detector 20 is measured as a function of the central wavelength.

Spectral analysis, in particular a Fourier transform, of the signal from detector 20 can then be used for generating the reflection values of eye 30 along axis z for the given A-scan.

This type of OCT measurement is known to the skilled person, and it is e.g. described in EP 3572765 and the references cited therein.

The device further comprises a control unit 32, which may e.g. be provided with a microprocessor 34 and with a memory 36 as well as with a display 38. Memory 36 may hold the data as well as the program instructions required for carrying out the steps of the present method. Display 38 may e.g. be used for showing the data determined thereby and in particular for displaying any images derived by means of the techniques described herein.

The device of Fig. 1 further comprises a fluorescence detector 40 for measuring time-resolved fluorescence data of fluorescent light returning from eye 30.

Fluorescence detector 40 comprises a light source 42 generating a probe beam 44. Advantageously, light source 42 is a pulsed light source generating short light pulses, in particular having a length of no more than 100 ps.

Probe beam 44 is sent through a first dichroic mirror 45 onto a beam combiner 46, where it is made collinear with measurement beam 28 of OCT interferometer 8.

In one embodiment, beam combiner 46 may e.g. be a second dichroitic beam splitter, which reflects probe beam 44 as well as the fluorescent light from the eye but transmits measurement beam 28, or vice versa.

Advantageously, probe beam 44 is sent, together with measurement beam 28, through the scanner mirrors 24a, 24b and objective lenses 26a, 26b.

Probe beam 44 advantageously has substantially the same focal point location, in particular within +/- 5 mm, advantageously within +/- 1 mm, as measurement beam 28 (in this context, "focal point location" designates the location of the focal point along direction z). This provides improved spatial synchronization between the fluorescence lifetime measurements and the OCT measurements.

The wavelength of probe beam 44 is selected to generate fluorescence in eye 30. Advantageously, this wavelength is in the range of 360 - 500 nm, advantageously at 470 nm $\pm$20 nm (for lipofuscin excitation) or 440 nm $\pm$20 nm (for A2E excitation), even though excitation outside this range may also be possible, often with reduced efficiency. The fluorescent light may be in the range of 500 - 700 nm, e.g. centered at about 600 - 610 nm for lipofuscin fluorescence or 565 - 575 nm for A2E fluorescence. The wavelength of the measurement beam 28 from interferometer 8 is e.g. in the range of 980 - 1150 nm or 700 - 900 nm, which allows to spectrally separate the OCT and fluorescence measurements.

Fluorescent light generated in the structures of the eye of probe beam 44 is, in part, fed back through objective lenses 26b, 26a, mirrors 24a, 24b and beam combiner 46 to arrive at first dichroic mirror 45.

Dichroic mirror 45 is e.g. designed such that it transmits the light of probe beam 44 but reflects the fluorescent light from the eye. Hence, the fluorescent light is reflected into a light detector 48.

The signal from light detector 48 is fed into a lifetime analyzer 50, which may form part of control unit 32. Lifetime analyzer 50 is structured and adapted to measure one or more parameters of the delay process of the fluorescent light as it will be described in more detail below.

Advantageously, measurement beam 28 as well as probe beam 44 pass focus controller 52, which allows to commonly adjust the focal point location of both measurement systems (OCT and lifetime spectroscopy). In particular, focus controller 52 is adapted to vary the focal point location over an effective optical distance (i.e. distance multiplied by effective refractive index of the regions passed by the beams) of at least 30 mm, in particular of at least 40 mm, such that the focal point location can be set into the retina as well as into the lens of eye 30.

The scanning mirrors 24a, 24b form scanning optics for commonly deflecting probe beam 44 and measurement beam 28 into directions x, y perpendicular to the optical axis z of the microscope device. This allows, as mentioned, to spatially synchronize the two measurements.

*Fluorescence Lifetime Measurements*

Fluorescent lifetime may e.g. be measured by sending a short pulse of probe light into the eye and performing a time-resolved measurement of the fluorescent response, i.e. of the raw fluorescent data (for alternative methods, see "Notes" below). In general, the fluorescent response as a function of time t will be a sum of exponential decays. This, for example is the fluorescent response $I_{FL}(t)$ of the lens of the eye:

$$I_{FL}(t) = \sum_{i=1}^{n} A_{L,i} \exp(-t/\tau_{L,i}) \qquad (1)$$

$A_i$ are the characteristic amplitudes and $\tau_i$ the decay times of the *n* involved fluorescent processes.

Fluorescence detector 40 is adapted to determine at least part of these amplitudes and decay times.

To do so, fluorescence detector 40 may e.g. be designed to carry out time-correlated single-photon counting (TCSPC). This widely used method involves sending several pulses into the eye and recording the responses using a fast single-photon detector. After enough recorded events, a histogram of the number of events across all the recorded time points is calculated, and then the amplitudes $A_i$ and the decay times $\tau_i$ of Eq. (1) are determined by curve fitting.

Advantageously, though, fluorescence detector 40 is adapted to carry out an "analog mean delay" measurement as e.g. described in in Moon et al., Optics Express 17(4), 2834 - 2849, US2019310198, and US2020088638. This method works with larger light intensities and is therefore faster than TCSPC.

*Lens Contribution Compensation*

In one embodiment, a fluorescence lifetime measurement is first performed on the lens of the eye by setting focus controller 52 to focus OCT measurement beam 28 as well as fluorescence probe beam 44 onto the lens of the eye, e.g. the center of the lens. This reduces the amount of fluorescent light returned to detector 48 from other structures of the eye.

The response from the lens takes the form of Eq. (1).

In a second step, focus controller 52 is set to focus OCT measurement beam 28 as well as fluorescence probe beam 44 onto the retina, and a scan along directions x and y (perpendicular to direction z) is carried out, which yields a response (raw fluorescent data) as a function of location *x, y* as follows:

$$\tilde{I}_{FR}(x,y,t) = \sum_{i=1}^{n} \tilde{A}_{R,i} \exp(-t/\tilde{\tau}_{R,i}) \qquad (2)$$

Here, $\tilde{A}_{R,i}$ and $\tilde{\tau}_{R,i}$ denote the amplitudes and decay times of a superposition of the fluorescent processes in the retina and the lens.

In order to isolate the contribution of the processes in the retina only, corrected fluorescence data $I_{FR}(x,y,t)$ is calculated from the raw fluorescent data $\tilde{I}_{FR}(x,y,t)$ as follows:

$$I_{FR}(x,y,t) = \tilde{I}_{FR}(x,y,t) - \alpha I_{FL}(t) = \sum_{i=1}^{n} A_{R,i,x,y} \exp(-t/\tau_{R,i,x,y}) \qquad (3)$$

Here, $I_{FL}(t)$ is the fluorescent response measured from the lens, $\alpha$ is correction factor, and $\alpha I_{FL}(t)$ is an estimate of the contribution of the lens fluorescence to the raw data. $A_{R,i,x,y}$ and $\tau_{R,i,x,y}$ are the amplitudes and decay times of the retina fluorescence as a function of the coordinates x, y.

Correction factor $\alpha$ may be as follows:

a) It may be a constant, which is e.g. estimated by the device manufacturer. For example, it may be estimated by ray tracing calculus while modeling the amount of lens fluorescence arriving on detector 48 when probe beam 44 is focused on the retina and using the approximation that the contribution of the lens fluorescence to the raw fluorescent data is substantially independent of the coordinates *x, y*. In this case, the "estimated fluorescence data" of the lens is the same for different locations *x, y*.

b) It may be selected depending on the thickness, volume, or shape of the lens or on the distance between the lens and the retina. These parameters can readily be measured by OCT.

c) It may be dependent on fluorescence inhomogeneities of the lens due to an inhomogeneous composition of the lens. In this case, the lens fluorescence $I_{FL}(t, x', y')$ is measured, in the first step above, by scanning it as a function of coordinates *x', y'* and directing probe beam 44 to different parts *x', y'* of the lens.

In cases b) and, in particular, c), the estimated of the contribution of the lens fluorescence is a function of coordinates *x, y*. Ray tracing calculus can be used to estimate this function. In other words, in this case the "estimated fluorescence data" of the lens is different for at least two different locations x, y. Such ray tracing may e.g. use the thickness, extension, volume or position of the second structure.

In the example above, it is assumed that the fluorescence lifetime of the retina is to be measured, and the contribution of lens fluorescence is to be eliminated. In more general terms, the fluorescence lifetime of a first structure of the eye is to be measured and the contribution of the fluorescence of a second structure of the eye is to be eliminated. For example, the structure of interest (the "first structure") may also be the lens and the structure whose contribution is to be eliminated (the "second structure") may be the retina. Other possible first and second structures include the cornea or the vitreous body of the eye.

In Eq. (1) above it is assumed that, when focusing on the lens, the contribution of retina fluorescence can be neglected. If this is not the case, Eq. (1) can be modified to include a contribution from the retina fluorescence. This contribution may e.g. be assumed to be the integral of $I_{FR}(x,y,t)$ over *x* and *y* scaled by a second correction factor $\alpha'$. The second correction factor $\alpha'$ can e.g. be assumed to be constant and be estimated by the device manufacturer using ray tracing calculus on typical eye model data.

*Retina Thickness Compensation*

When scanning the fluorescence of a structure along x- and y-, the amount of fluorescence of the structure is often proportional to the thickness $t_{x,y}$ of the structure along direction z.

Hence, it can be of interest to calculate a normalized fluorescence parameter, such as normalized fluorescent amplitudes $A'_{R,i,x,y}$, by normalizing the uncorrected fluorescence parameter with said thickness, i.e.

$$A'_{R,i,x,y} = A_{R,i,x,y} / t_{x,y}. \tag{4}$$

Such a normalized fluorescence parameter may e.g. describe a composition or activity of a given part of a structure more reliably than its non-normalized counterpart. This is particularly true for the retina, for which the thickness varies as a function of x and y, but degenerative defects can better be determined from the normalized parameter.

The thickness $t_{x,y}$ can be readily measured by means of interferometer 8, i.e. by means of an OCT measurement.

The thickness $t_{x,y}$ can e.g. be the retinal pigment epithelium (RPE) thickness of the retina, but other thickness parameters may be used as well.

*Motion Compensation*

When carrying out several fluorescence lifetime measurements over an extended time period, such as over at least 1 ms, motion artifacts of the eye may render it difficult to correlate the time response of the measurements. For example, when the patient moves eye 30 closer to the device, the fluorescent signals will be quicker to arrive at detector 48.

This may e.g. the case during repetitive TCSPC measurements or while scanning a structure of the eye along the x- and y-coordinates.

With the present device, OCT measurements can be used to monitor the distance between the eye and the device, and the measured changes in the distance can be used to compensate a time-offset in the fluorescence data.

For example, the distance to the "first" or "second" structure as defined above can be monitored. Alternatively, the distance along z between the device and the cornea or any other part of the may be monitored.

If, for example, $F1(t_1)$ designates time response of a first fluorescent measurement and $F2(t_2)$ designates the response of a subsequent second fluorescent measurement, with time $t_1$ and $t_2$ being time relative to the probe light pulse, the two responses may be shifted in time if the eye has moved along z.

For example, if the eye has moved away from the device along z, between the two measurements, by a distance *d*, the second measurement can be offset by *2d* for comparing it with the first measurement, i.e. *F2(t + 2d)* has the same time frame as *F1(t)*, e.g. for superimposing event times in two TCSPC measurements.

Alternatively to (or in addition to) compensating changes in distance, the OCT measurements can be used to monitor that the eye is in a suitable range for the fluorescence measurements. In this case, a fluorescence measurement is enabled only if the distance is in a given range.

*Intra-Eye Delay Compensation*

The temporal resolution of fluorescence lifetime measurements may reach an order of magnitude of e.g. 10 ps. In this time, light travels a distance of 3 mm in vacuum.

Hence, the spacing between the structures of the eye may give rise to noticeably different delays in the fluorescent response from the various structures as measured by detector 48. For example, the fluorescent light from the lens may arrive at detector 48 several 10 ps before the fluorescent light from the retina.

To compensate for this, for example, Eq. (3) may be replaced with

$$I_{FR}(x, y, t) = \tilde{I}_{FR}(x, y, t) - \alpha I_{FL}(t + 2Dn/c) \tag{5}$$

Here, *D* is the (average) distance between the first and the second structure, *n* is the (average) refractive index of the eye between the two structures, and *c* is the speed of light. Eq. (5) takes into account that the fluorescence from the second structure (the lens, in this case), will arrive at detector 48 earlier than the fluorescence of the first structure (the retina, in this case), namely by the time it takes for the light to travel from the second to the first structure and back, i.e. by *2Dn/c.*

The distance *D* can be a typical distance between the two structures in the human eye. Advantageously, though, distance *D* is measured by means of OCT with interferometer 8.

Hence, in more general terms, the method may comprise the step of offsetting the estimated fluorescence data of the second structure may in time as a function of the distance *D* between the first and the second structure before it is combined with the raw fluorescence data in order to calculate the corrected fluorescence data. Advantageously, the distance $D$ is measured by means of the OCT measurements.

*Notes*

As shown above, combining an OCT interferometer and a fluorescence detector in a single device provides numerous advantages. A further advantage of such a system is the fact that the space-resolved lifetime fluorescence data along x and y can be easily mapped to structures in the eye determined by OCT. Such mapping can e.g. be used to create superimposed images of structural and fluorescent features and/or to categorize structures.

On the other hand, as described above, it is possible to correct the fluorescence lifetime raw data of a first structure by estimating the fluorescence response of a second structure interfering with the experiment and by using the estimated fluorescence data of the second structure for generating corrected fluorescence data for the first structure. As shown, this technique can be advantageously combined with OCT measurements from the same device, but it can also be used with devices that do not provide OCT capability.

As it has been mentioned, fluorescent lifetime may be determined in the time domain by sending a short pulse of probe light into the eye and performing a time-resolved measurement of the fluorescent response. Alternatively, though, the measurements may be carried out in the frequency domain, see e.g. Shim et al. in Journal of the Korean Physical society, 49, pp. S647 - S651. In this case, the light source is pulsed or modulated at a high frequency. The amplitude and phase shift of the fluorescent signal may be measured for different frequencies, which allows to retrieve one or more of the characteristic amplitudes $A_i$ and decay times $\tau_i$. Once these parameters are known, the methodology above can be used to compensate for the contribution of the "second structure", such as the lens, in the raw fluorescence data.

The techniques described here can be used with any kind of OCT, in particular with time-domain OCT as well as frequency-domain OCT. Frequency-domain OCT, and in particular swept-source OCT, is, however, advantageous for its ability to obtain an A-scan quickly.

In the above examples, it is assumed that the response of fluorescence detector 48 is instantaneous and the length of the pulse of light source 42 negligible. If that is not the case, the signals at detector 48 are convolutions of the detector response, the light pulse shape, and the exponential fluorescent decays, and the functions to be fitted need to be adapted accordingly as known to the skilled person.

While there are shown and described presently preferred embodiments of the invention, it is to be distinctly understood that the invention is not limited thereto but may be otherwise variously embodied and practiced within the scope of the following claims.

## Claims

1.  A method for carrying out fluorescence lifetime microscopy on a first structure of an eye comprising the steps of

    sending, by means of a probe light source (42), a probe beam (44) into the eye, wherein said probe beam (44) interacts with at least said first and a second structure of said eye, with said first and said second structure being spaced apart from each other,
    measuring, by means of a fluorescence detector (48), time-resolved raw fluorescence data returning from said eye,
    wherein the method is **characterized by**
    calculating, using a control unit (32), time-resolved corrected fluorescence data for said first structure from said raw fluorescence data and from estimated fluorescence data originating from said second structure.

2.  The method of claim 1 comprising the step of performing a first time-resolved fluorescence measurement with said probe beam (44) focused on said first structure, wherein said raw fluorescence data is derived from said first measurement.

3.  The method of claim 2 comprising the step of performing a second time-resolved fluorescence measurement with said probe beam (44) focused on said second structure, wherein said estimated fluorescence data is derived from said second measurement.

4.  The method of any of the preceding claims comprising the step of measuring said raw fluorescence data as a function of at least two locations in said first structure.

5.  The method of claim 4 wherein the estimated fluorescence data is the same for the at least two locations.

6. The method of claim 4 wherein the estimated fluorescence data is different for the at least two locations.

7. The method of the claims 3 and 6 comprising the step of obtaining spatially resolved estimated fluorescence data by performing a plurality of the second time-resolved fluorescence measurements with the probe beam (44) focused on different parts of said second structure.

8. The method of any of the claims 6 or 7 comprising the step of determining, in particular by means of ray tracing calculations, two parts of the second structure that said probe beam (44) interacts with when being focused on said two locations of the first structure.

9. The method of any of the preceding claims comprising the step of offsetting the estimated fluorescence data of the second structure in time as a function of a distance (D) between the first and the second structure before it is combined with the raw fluorescence data in order to calculate the corrected fluorescence data.

10. The method of any of the preceding claims, wherein a device equipped for OCT and time-resolved fluorescence measurements is used for said fluorescence lifetime microscopy, and wherein said method comprises the step of carrying out OCT measurements on said first and/or second structures with said device.

11. The method of claim 10 wherein said OCT measurements are carried out by means of a measurement beam (28) collinear to said probe beam (44).

12. The method of any of the claims 10 or 11 comprising the step of using OCT data obtained from said OCT measurements for calculating said estimated fluorescence data,
and in particular wherein at least one parameter descriptive of one or more of the following parameters of the second structure is used for calculating the fluorescence data: a thickness of the second structure along an axis of the eye, a volume of the second structure, an extension of the second structure perpendicular to the axis of the eye, or a position of the second structure.

13. The method of any of the claims 10 to 12 comprising the steps of

measuring a distance, by means of said OCT measurements, between a part of the eye and said device and using said distance for compensating a time-offset in at least one of said raw fluorescence data, said estimated fluorescence data, and said corrected fluorescence data,
and in particular wherein said part of the eye is said first structure or said second structure.

14. The method of claim 13 comprising the steps of

measuring said distance for at least two subsequent fluorescence lifetime measurements and
mutually offsetting, in time, the at least two fluorescence lifetime measurements as a function of the change of said distance in between the at least two fluorescence measurements and/or enabling a fluorescence lifetime measurement only if said distance is in a given range.

15. The method of any of the claims 10 to 14 comprising the step of calculating normalized fluorescence parameter for several locations of said first structure by normalizing the corrected fluorescence data at said locations with a thickness parameter of said first structure at said locations, wherein said thickness parameter is measured with an OCT measurement.

16. The method of claim 9 and of any of the claims 10 to 15 wherein said distance (D) between the first and the second structure is measured by means of said OCT measurements.

17. The method of any of the preceding claims, wherein

the first structure is a retina of the eye and/or the second structure is a lens of the eye or
the second structure is a retina of the eye and/or the first structure is a lens of the eye.

18. A microscope device for carrying out fluorescence lifetime microscopy on a first structure of an eye comprising

a probe light source (42) for sending a probe beam (44) into the eye,

a fluorescence detector (48) for measuring time-resolved raw fluorescence data from fluorescent light returning from said eye, and
a control unit (32) adapted to carry out the steps of the method of any of the preceding claims.

19. The device of claim 18 wherein the fluorescence detector is adapted to derive fluorescence lifetime parameters from the time-resolved fluorescence data, and wherein the device further comprises
an interferometer (8) for sending a measurement beam (28) into the eye and carrying out optical coherence tomography on light of said measurement beam (28) reflected from structures within said eye.

20. The device of claim 19 further comprising a beam combiner (46) arranged to collinearly combine said probe beam (44) and the measurement beam (28).

21. The device of any of the claims 19 or 20 further comprising scanning optics (24a, 24b) for commonly deflecting the probe beam (44) and the measurement beam (28) into directions (x, y) perpendicular to an optical axis (z) of the device.

22. The device of any of the claims 19 to 21 further comprising a control unit (32) adapted to carry out the steps of

measuring a distance, by means of said OCT measurements, between a part of the eye and said device and using said distance for compensating a time-offset in said fluorescence data and/or to enable a fluorescence measurement only if said distance is in a given range.

23. The device of claim 22 wherein said control unit is further adapted to carry out the steps of

measuring said distance for at least two subsequent fluorescence measurements and
mutually offsetting, in time, the at least two fluorescence measurements as a function of the change of said distance in between the at least two fluorescence measurements.

24. The device of any of the claims 19 to 23 further comprising a focus controller (52) for commonly adjusting a focal point location of both said measurement beam (28) and said probe beam (44).

25. The device of any of the claims 19 to 24 wherein a focal point location of the probe beam (44) and the measurement beam (28) are within +/- 5 mm, in particular within +/- 1 mm from each other.

**Patentansprüche**

1. Verfahren zur Durchführung von Fluoreszenzzeit-Mikroskopie an einer ersten Struktur eines Auges, das die folgenden Schritte umfasst

Senden eines Prüfstrahls (44) mittels einer Prüflichtquelle (42) in das Auge, wobei der Prüfstrahl (44) mit mindestens der ersten und einer zweiten Struktur des Auges in Wechselwirkung tritt, wobei die erste und die zweite Struktur voneinander beabstandet sind,
Messung von vom Auge zurückkehrenden zeitaufgelösten Fluoreszenz-Rohdaten mit Hilfe eines Fluoreszenzdetektors (48),
wobei das Verfahren **gekennzeichnet ist durch**
Berechnen von zeitaufgelösten korrigierten Fluoreszenzdaten für die erste Struktur aus den Fluoreszenz-Rohdaten und aus geschätzten Fluoreszenzdaten, die von der zweiten Struktur stammen, unter Verwendung einer Steuereinheit (32).

2. Verfahren nach Anspruch 1, umfassend den Schritt der Durchführung einer ersten zeitaufgelösten Fluoreszenzmessung mit auf die erste Struktur fokussiertem Prüfstrahl (44), wobei die Fluoreszenz-Rohdaten aus der ersten Messung abgeleitet werden.

3. Verfahren nach Anspruch 2, umfassend den Schritt der Durchführung einer zweiten zeitaufgelösten Fluoreszenzmessung mit auf die zweite Struktur fokussiertem Prüfstrahl (44), wobei die geschätzten Fluoreszenzdaten aus der zweiten Messung abgeleitet werden.

**4.** Verfahren nach einem der vorhergehenden Ansprüche, umfassend den Schritt des Messens der Fluoreszenz-Rohdaten als Funktion von mindestens zwei Stellen in der ersten Struktur.

**5.** Verfahren nach Anspruch 4, wobei die geschätzten Fluoreszenzdaten für die mindestens zwei Orte gleich sind.

**6.** Verfahren nach Anspruch 4, wobei die geschätzten Fluoreszenzdaten für die mindestens zwei Orte unterschiedlich sind.

**7.** Verfahren nach den Ansprüchen 3 und 6, umfassend den Schritt des Erhaltens von räumlich aufgelösten geschätzten Fluoreszenzdaten durch Durchführen einer Vielzahl von zweiten zeitaufgelösten Fluoreszenzmessungen, wobei der Prüfstrahl (44) auf verschiedene Teile der zweiten Struktur fokussiert wird.

**8.** Verfahren nach einem der Ansprüche 6 oder 7, umfassend den Schritt des Bestimmens, insbesondere mittels Beam-Tracing-Berechnungen, zweier Teile der zweiten Struktur, mit denen der Prüfstrahl (44) in Wechselwirkung tritt, wenn er auf die beiden Stellen der ersten Struktur fokussiert wird.

**9.** Das Verfahren nach einem der vorhergehenden Ansprüche, umfassend den Schritt des zeitlichen Versatzes der geschätzten Fluoreszenzdaten der zweiten Struktur als Funktion eines Abstands (D) zwischen der ersten und der zweiten Struktur, bevor sie mit den Fluoreszenz-Rohdaten kombiniert werden, um die korrigierten Fluoreszenzdaten zu berechnen.

**10.** Verfahren nach einem der vorhergehenden Ansprüche, wobei für die Fluoreszenz-Zeit-Mikroskopie eine Vorrichtung verwendet wird, die für OCT und zeitaufgelöste Fluoreszenzmessungen ausgerüstet ist, und wobei das Verfahren den Schritt der Durchführung von OCT-Messungen an den ersten und/oder zweiten Strukturen mit der Vorrichtung umfasst.

**11.** Verfahren nach Anspruch 10, bei dem die OCT-Messungen mit Hilfe eines Messstrahls (28) durchgeführt werden, der kollinear zu dem Prüfstrahl (44) verläuft.

**12.** Verfahren nach einem der Ansprüche 10 oder 11, umfassend den Schritt der Verwendung von OCT-Daten, die aus den OCT-Messungen erhalten wurden, zur Berechnung der geschätzten Fluoreszenzdaten, und insbesondere wobei mindestens ein Parameter, der einen oder mehrere der folgenden Parameter der zweiten Struktur beschreibt, zur Berechnung der Fluoreszenzdaten verwendet wird: eine Dicke der zweiten Struktur entlang einer Achse des Auges, ein Volumen der zweiten Struktur, eine Ausdehnung der zweiten Struktur senkrecht zur Achse des Auges oder eine Position der zweiten Struktur.

**13.** Verfahren nach einem der Ansprüche 10 bis 12, das die folgenden Schritte umfasst

Messung des Abstands zwischen einem Teil des Auges und der Vorrichtung mit Hilfe der OCT-Messungen und Verwendung des Abstands zur Kompensation eines Zeitversatzes in mindestens einem der Fluoreszenz-Rohdaten, der geschätzten Fluoreszenzdaten und der korrigierten Fluoreszenzdaten, und insbesondere, wenn der Teil des Auges die erste Struktur oder die zweite Struktur ist.

**14.** Das Verfahren nach Anspruch 13, umfassend die folgenden Schritte

Messung des Abstands für mindestens zwei aufeinander folgende Messungen der Fluoreszenzzeit und relatives zeitliches Versetzen der mindestens zwei Fluoreszenzzeitmessungen in Abhängigkeit von der Änderung des Abstands zwischen den mindestens zwei Fluoreszenzmessungen und/oder Ermöglichen einer Fluoreszenzzeitmessung nur dann, wenn der Abstand in einem bestimmten Bereich liegt.

**15.** Verfahren nach einem der Ansprüche 10 bis 14, umfassend den Schritt des Berechnens normalisierter Fluoreszenzparameter für mehrere Stellen der ersten Struktur durch Normalisieren der korrigierten Fluoreszenzdaten an diesen Stellen mit einem Dickenparameter der ersten Struktur an diesen Stellen, wobei der Dickenparameter mit einer OCT-Messung gemessen wird.

**16.** Verfahren nach Anspruch 9 und nach einem der Ansprüche 10 bis 15, wobei der Abstand (D) zwischen der ersten und der zweiten Struktur mit Hilfe der OCT-Messungen gemessen wird.

**17.** Verfahren nach einem der vorhergehenden Ansprüche, wobei

die erste Struktur ist eine Netzhaut des Auges und/oder die zweite Struktur ist eine Linse des Auges oder die zweite Struktur ist eine Netzhaut des Auges und/oder die erste Struktur ist eine Linse des Auges.

**18.** Mikroskopische Vorrichtung zur Durchführung von Fluoreszenzzeit-Mikroskopie an einer ersten Struktur eines Auges mit

eine Prüflichtquelle (42) zum Senden eines Prüfstrahls (44) in das Auge,
einen Fluoreszenzdetektor (48) zur Messung von zeitaufgelösten Fluoreszenz-Rohdaten aus dem von dem Auge zurückkehrenden Fluoreszenzlicht, und
eine Steuereinheit (32), die dazu ausgestaltet ist, die Schritte des Verfahrens nach einem der vorhergehenden Ansprüche auszuführen.

**19.** Vorrichtung nach Anspruch 18, wobei der Fluoreszenzdetektor dazu ausgestaltet ist, aus den zeitaufgelösten Fluoreszenzdaten Fluoreszenzzeitparameter abzuleiten, und wobei die Vorrichtung ferner Folgendes umfasst ein Interferometer (8) zum Senden eines Messstrahls (28) in das Auge und zum Durchführen einer optischen Kohärenztomographie am Licht des Messstrahls (28), das von Strukturen innerhalb des Auges reflektiert wird.

**20.** Vorrichtung nach Anspruch 19, umfassend ferner einen Strahlkombinierer (46), der so angeordnet ist, dass er den Prüfstrahl (44) und den Messstrahl (28) kollinear kombiniert.

**21.** Vorrichtung nach einem der Ansprüche 19 oder 20, umfassend ferner eine Abtastoptik (24a, 24b) zur gemeinsamen Ablenkung des Prüfstrahls (44) und des Messstrahls (28) in Richtungen (x, y), die senkrecht zu einer optischen Achse (z) der Vorrichtung stehen.

**22.** Vorrichtung nach einem der Ansprüche 19 bis 21, umfassend ferner eine Steuereinheit (32), die dazu ausgestaltet ist, die folgenden Schritte auszuführen

Messung des Abstands zwischen einem Teil des Auges und der Vorrichtung mit Hilfe der OCT-Messungen und Verwendung der Entfernung zur Kompensation eines Zeitversatzes in den Fluoreszenzdaten und/oder zur Ermöglichung einer Fluoreszenzmessung nur dann, wenn die Entfernung in einem bestimmten Bereich liegt.

**23.** Vorrichtung nach Anspruch 22, wobei die Steuereinheit ferner zum Ausführen der folgenden Schritte ausgestaltet ist:

Messung des genannten Abstands für mindestens zwei aufeinander folgende Fluoreszenzmessungen und gegenseitige zeitliche Verschiebung der mindestens zwei Fluoreszenzmessungen in Abhängigkeit von der Änderung des Abstands zwischen den mindestens zwei Fluoreszenzmessungen.

**24.** Vorrichtung nach einem der Ansprüche 19 bis 23, umfassend ferner eine Fokussteuerung (52) zum gemeinsamen Einstellen einer Brennpunktposition sowohl des Messstrahls (28) als auch des Prüfstrahls (44).

**25.** Vorrichtung nach einem der Ansprüche 19 bis 24, wobei ein Brennpunkt des Prüfstrahls (44) und des Messstrahls (28) innerhalb von +/- 5 mm, insbesondere innerhalb von +/- 1 mm, voneinander entfernt sind.

**Revendications**

**1.** Un procédé pour exécuter une microscopie à fluorescence sur une première structure d'un œil, comprenant les étapes suivantes

envoyer, au moyen d'une source de lumière de sonde (42), un faisceau de sonde (44) dans l'œil, dans lequel ledit faisceau de sonde (44) interagit avec au moins ladite première et une seconde structure dudit œil, ladite première et ladite seconde structure étant espacées l'une de l'autre,
mesurer, au moyen d'un détecteur de fluorescence (48), des données brutes de fluorescence résolues dans le temps provenant de l'œil,
dans lequel le procédé est **caractérisée par**
calculer, en utilisant une unité de commande (32), des données de fluorescence corrigées résolues dans le temps pour ladite première structure à partir desdites données de fluorescence brutes et des données de fluorescence estimées provenant de ladite seconde structure.

2. Le procédé selon la revendication 1, comprenant l'étape consistant à effectuer une première mesure de fluorescence résolue dans le temps avec ledit faisceau de sonde (44) focalisé sur ladite première structure, dans lequel lesdites données de fluorescence brutes sont dérivées de ladite première mesure.

3. Le procédé selon la revendication 2, comprenant l'étape consistant à effectuer une deuxième mesure de fluorescence résolue dans le temps avec ledit faisceau de sonde (44) focalisé sur ladite deuxième structure, dans laquelle les données de fluorescence estimées sont dérivées de ladite deuxième mesure.

4. Le procédé selon l'une des revendications précédentes, comprenant l'étape de mesure de ces données brutes de fluorescence en fonction d'au moins deux emplacements dans la première structure.

5. Le procédé selon la revendication 4, dans lequel les données de fluorescence estimées sont les mêmes pour les au moins deux emplacements.

6. Le procédé selon la revendication 4, dans lequel les données de fluorescence estimées sont différentes pour les au moins deux emplacements.

7. Le procédé selon les revendications 3 et 6, comprenant l'étape consistant à obtenir des données de fluorescence estimées résolues dans l'espace en effectuant une pluralité de secondes mesures de fluorescence résolues dans le temps avec le faisceau de sonde (44) focalisé sur différentes parties de ladite seconde structure.

8. Le procédé selon l'une quelconque des revendications 6 ou 7, comprenant l'étape consistant à déterminer, en particulier au moyen de calculs de traçage de rayons, deux parties de la seconde structure avec lesquelles ledit faisceau de sonde (44) interagit lorsqu'il est focalisé sur lesdits deux emplacements de la première structure.

9. Le procédé selon l'une quelconque des revendications précédentes, comprenant l'étape de décalage dans le temps des données de fluorescence estimées de la seconde structure en fonction d'une distance ($D$) entre la première et la seconde structure avant qu'elles ne soient combinées avec les données de fluorescence brutes afin de calculer les données de fluorescence corrigées.

10. Le procédé selon l'une quelconque des revendications précédentes, dans lequel un dispositif équipé pour les mesures OCT et de fluorescence résolue dans le temps est utilisé pour ladite microscopie à durée de vie de fluorescence, et dans lequel ledit procédé comprend l'étape consistant à effectuer des mesures OCT sur lesdites première et/ou seconde structures avec ledit dispositif.

11. Le procédé selon la revendication 10, dans lequel les mesures OCT sont effectuées au moyen d'un faisceau de mesure (28) colinéaire au faisceau de sonde (44).

12. Le procédé selon l'une quelconque des revendications 10 ou 11, comprenant l'étape consistant à utiliser les données OCT obtenues à partir desdites mesures OCT pour calculer lesdites données de fluorescence estimées, et en particulier dans lequel au moins un paramètre descriptif d'un ou plusieurs des paramètres suivants de la deuxième structure est utilisé pour calculer les données de fluorescence : une épaisseur de la deuxième structure le long d'un axe de l'œil, un volume de la deuxième structure, une extension de la deuxième structure perpendiculaire à l'axe de l'œil, ou une position de la deuxième structure.

13. Le procédé selon l'une quelconque des revendications 10 à 12, comprenant les étapes suivantes

mesurer une distance, au moyen desdites mesures OCT, entre une partie de l'œil et ledit dispositif et utiliser cette distance pour compenser un décalage temporel dans au moins l'une des données de fluorescence brutes, des données de fluorescence estimées et des données de fluorescence corrigées, et en particulier dans lequel ladite partie de l'œil est ladite première structure ou ladite seconde structure.

14. Le procédé selon la revendication 13, comprenant les étapes suivantes

mesurer ladite distance pour au moins deux mesures ultérieures de la durée de vie de la fluorescence et

décaler mutuellement, dans le temps, les au moins deux mesures de durée de vie de fluorescence en fonction de la variation de ladite distance entre les au moins deux mesures de fluorescence et/ou n'autoriser une mesure de durée de vie de fluorescence que si ladite distance se situe dans une plage donnée.

15. Le procédé selon l'une quelconque des revendications 10 à 14, comprenant l'étape de calcul du paramètre de fluorescence normalisé pour plusieurs emplacements de ladite première structure en normalisant les données de fluorescence corrigées auxdits emplacements avec un paramètre d'épaisseur de ladite première structure auxdits emplacements, ledit paramètre d'épaisseur étant mesuré à l'aide d'une mesure OCT.

16. Le procédé selon la revendication 9 et selon l'une quelconque des revendications 10 à 15, dans lequel la distance ($D$) entre la première et la seconde structure est mesurée au moyen de ces mesures OCT.

17. Le procédé selon l'une quelconque des revendications précédentes, dans lequel

la première structure est une rétine de l'œil et/ou la seconde structure est un cristallin de l'œil ou
la deuxième structure est une rétine de l'œil et/ou la première structure est un cristallin de l'œil.

18. Un dispositif microscopique permettant d'effectuer une microscopie à durée de vie de fluorescence sur une première structure d'un oeil, comprenant

une source de lumière de sonde (42) pour envoyer un faisceau de sonde (44) dans l'oeil,
un détecteur de fluorescence (48) pour mesurer les données brutes de fluorescence résolues dans le temps à partir de la lumière fluorescente revenant de l'œil, et
une unité de commande (32) adaptée pour exécuter les étapes selon le procédé selon l'une quelconque des revendications précédentes.

19. Le dispositif selon la revendication 18, dans lequel le détecteur de fluorescence est adapté pour dériver les paramètres de durée de vie de la fluorescence à partir des données de fluorescence résolues dans le temps, et dans lequel le dispositif comprend en outre
un interféromètre (8) pour envoyer un faisceau de mesure (28) dans l'œil et effectuer une tomographie par cohérence optique sur la lumière dudit faisceau de mesure (28) réfléchie par les structures à l'intérieur de l'œil.

20. Le dispositif selon la revendication 19, comprenant en outre un combineur de faisceaux (46) conçu pour combiner de manière colinéaire ledit faisceau de sonde (44) et le faisceau de mesure (28).

21. Le dispositif selon l'une quelconque des revendications 19 ou 20, comprenant en outre une optique de balayage (24a, 24b) pour dévier communément le faisceau de sonde (44) et le faisceau de mesure (28) dans des directions (x, y) perpendiculaires à un axe optique (z) du dispositif.

22. Le dispositif selon l'une quelconque des revendications 19 à 21, comprenant en outre une unité de commande (32) adaptée pour effectuer les étapes suivantes

mesurer une distance, au moyen desdites mesures OCT, entre une partie de l'œil et ledit dispositif et
utiliser cette distance pour compenser un décalage temporel dans les données de fluorescence et/ou pour permettre une mesure de fluorescence uniquement si cette distance se situe dans une plage donnée.

23. Le dispositif selon la revendication 22, dans lequel l'unité de commande est en outre adaptée pour effectuer les étapes suivantes

mesurer ladite distance pour au moins deux mesures de fluorescence ultérieures et
décaler mutuellement dans le temps les au moins deux mesures de fluorescence en fonction de la variation de ladite distance entre les au moins deux mesures de fluorescence.

24. Le dispositif selon l'une quelconque des revendications 19 à 23, comprenant en outre un contrôleur de focalisation (52) pour ajuster communément l'emplacement d'un point focal dudit faisceau de mesure (28) et dudit faisceau de sonde (44).

25. Le dispositif selon l'une des revendications 19 à 24, dans lequel le point focal du faisceau de sonde (44) et du faisceau de mesure (28) est situé à +/- 5 mm, en particulier à +/- 1 mm l'un de l'autre.

Fig. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3572765 A
- WO 2015072964 A
- WO 2019002256 A1
- US 2019310198 A
- US 2020088638 A

**Non-patent literature cited in the description**

- **MOON et al.** *Optics Express*, vol. 17 (4), 2834-2849
- **SHIM et al.** *Journal of the Korean Physical society*, vol. 49, S647-S651